# EUROPEAN PATENT APPLICATION

(11) **EP 4 371 419 A1**
(43) Date of publication of application: **22.05.2024**
(21) Application number: 22207622.6
(22) Date of filing: 15.11.2022
(51) Int. Cl.: A23L 33/15, A23L 33/175, A61K 8/44, A61K 31/198, A61K 31/401, A61P 43/00, A61Q 19/08, A23K 20/142, A23K 50/40

(54) **NON-THERAPEUTIC USE OF A COMPOSITION FOR MAINTAINING OR PROLONGING COLLAGEN HOMEOSTASIS IN A LIVING ORGANISM DURING AGING**

(71) Applicant: Avea Life AG, 6300 Zug ZG (CH)
(72) Inventor: Ewald, Collin, 8610 Uster (CH)
(74) Representative: Schaad, Balass, Menzl & Partner AG

(57) **Abstract**

The present invention relates to a non-therapeutic use of a composition for maintaining or prolonging collagen homeostasis in a living organism and/or for extending the healthspan and/or lifespan of a living organism, the composition containing glycine, proline and hydroxyproline in free form.

## Description

The present invention relates to a non-therapeutic use of a composition for maintaining or prolonging collagen homeostasis in a living organism during aging, as well as to a nutritional supplement preparation for a mammal, in particular a human individual.

In general, quality of life gradually decreases during aging of a living organism, such as a human individual. In particular, aging has been found to be the major factor for developing chronic diseases in an individual and is therefore subject of intensive research. The focus of this research is on the identification of interventions that counteract the aging-induced decrease in an individual's quality of life. Specifically, concepts are being investigated that have the potential to reduce aging-related morbidity and to increase an individual's lifespan and healthspan.

However, the biochemical mechanisms underlying aging and aging-related morbidity is not fully understood yet, and the development of efficient concepts and therapies is a challenging task.

Various technologies aiming at increasing longevity of organisms have been suggested in the art.

US2021052535A1 relates to a method for treatment or amelioration of aging or an aging-related condition negatively impacting longevity or quality of life by administering to a patient in need thereof one or more compounds selected from the group consisting of amino acids, peptides, carbohydrates, cofactors, vitamins, xenobiotics, lipids, and combinations thereof, or pharmaceutically acceptable salts, solvates, stereoisomers, or esters thereof.

The document focusses on pathological conditions negatively impacting longevity or quality of life, namely inflammation, insulin resistance, psoriasis, rheumatoid arthritis, fibrotic diseases, anemia, atherosclerosis or restenosis. In consideration of the variety conditions and of the almost infinite number of compounds, no specific data in support of a therapeutic activity are presented in this document.

US2015038547A1 discloses oral nutritional supplement products for the purpose of improvement of nutritional reserves containing L-proline as the sole active ingredient, in particular to promote longevity and/or to improve health and wellness.

However, also US 2015/0038547 is very unspecific in the underlying mechanisms but merely discusses the problem of amino acid shortage due to lack of adsorption of nutrition.

US5945330A reports on the discovery of two new human "longevity-assurance protein homologs" and the polynucleotides encoding them for providing new compositions useful in the diagnosis, prevention and treatment of disorders associated with aberrant regulation of cellular homeostasis and disorders associated with aging.

In the scientific literature, collagen supplementation has been reported to be sufficient to increase the lifespan of rats (see Liang J, Pei X, Zhang Z, Wang N, Wang J & Li Y (2010), The Protective Effects of Long-Term Oral Administration of Marine Collagen Hydrolysate from Chum Salmon on Collagen Matrix Homeostasis in the Chronological Aged Skin of Sprague-Dawley Male Rats. J Food Sci 75, H230-H238*,* and Liang J, Pei X-R, Wang N, Zhang Z-F, Wang J-B & Li Y (2010), Marine collagen peptides prepared from chum salmon (Oncorhynchus keta) skin extend the life span and inhibit spontaneous tumor incidence in Sprague-Dawley Rats, J Med Food 13, 757-70) .

Other authors have reported on the continuous decline of collagen and cell adhesion gene expression accompanied with an increase in matrix metalloproteinase expression to be a key signature of aging (Ewald, C. Y. (2020). The Matrisome during Aging and Longevity: A Systems-Level Approach toward Defining Matreotypes Promoting Healthy Aging. Gerontology, 66(3), 266-274*)*). Further, gene expression ontologies of extracellular matrix proteins (ECM) have been associated with healthy aging in humans (Zeng, L., Yang, J., Peng, S., Zhu, J., Zhang, B., Suh, Y., & Tu, Z. (2020). Transcriptome analysis reveals the difference between "healthy" and "common" aging and their connection with age-related diseases. Aging Cell, 19, e13121.)

Studies in C. *elegans* have further shown that collagen homeostasis is required and sufficient for longevity in this model organism (Ewald, C. Y., Landis, J. N., Abate, J. P., Murphy, C. T., & Blackwell, T. K. (2015). Dauer-independent insulin/IGF-1-signalling implicates collagen remodelling in longevity. Nature, 519, 97-101*).*

However, possible health benefits obtainable by collagen supplementation supporting better nutrition during aging and the respective underlying biochemical mechanisms are poorly understood. In particular, it is unknown which part of the collagen might promote longevity.

Most of the concepts suggested until now are based on supplementing naturally occurring collagen or collagen hydrolysate, primarily aiming at a repletion to counteract the collagen decline during aging. However, the acceptable daily intake of collagen or collagen hydrolysate by a human is limited. Also, collagens or collagen hydrolysates have to be broken down and are not adsorbed well, thus limiting the uptake of other (high-quality) proteins, which is in particular problematic in elderly people, who commonly take up insufficient amounts of protein. For native collagens being taken up, it takes more than 12 hours of digestion in the stomach and the small intestine, and a major part of undigested native collagen typically reaches the big intestine causing the formation of gases and bloating.

In addition, collagen or collagen hydrolysate used as a nutritional supplement as used in the rat model discussed in the scientific literature referred to above, is typically based on an animal collagen source, in particular fish or chicken, which is often not accepted by the consumer.

There is therefore a need for a compound or composition having potential benefits in terms of an organism's healthspan and lifespan but which does not have the disadvantages reported for collagen or collagen hydrolysate. However, the development of such a compound or composition has turned out to be complex, since the physiological mechanisms involved in increasing the healthspan and lifespan of an organism have not been elucidated fully and are still being investigated, as discussed above.

In consideration of the drawbacks of the known supplementation concepts mentioned above, the present invention aims at providing a composition for a non-therapeutic use, which can be prepared in a simple manner, and which allows to be administered to a living organism, and in particular to a human, in a dose effective for prolonging collagen homeostasis and/or for extending the healthspan and/or lifespan of a living organism.

The object of the present invention is solved by the composition of claim 1. Preferred embodiments of the invention are defined in the dependent claims.

According to claim 1, the present invention relates to a non-therapeutic use of a composition for maintaining or prolonging collagen homeostasis in a living organism and/or for extending the healthspan and/or lifespan of a living organism, the composition containing glycine, proline and hydroxyproline in free form.

The invention is based on the surprising finding that by administering to the organism the specific combination of amino acids defined above, collagen homeostasis of the organism can be maintained or prolonged and, ultimately, the organism's healthspan and/or the lifespan can be increased, as will be discussed in more detail in the following with regard to the experimental evidence (obtained in a C. *elegans* model as well as in human fibroblasts) in support of this finding:

By using a transcriptional reporter expressing green fluorescent protein (GFP) driven by the collagen col-144 promoter (Pcol-144::GFP), which expression progressively declined within 6-8 days of adulthood of C. *elegans,* it was observed by the present inventors that supplementing C. *elegans* with a combination of glycine, proline and hydroxyproline in free form resulted in a prolongation of endogenous col-144 expression during aging. (Regarding the use of Pcol-144::GFP as a suitable surrogate marker to predict longevity, reference is made to Statzer, C., Jongsma, E., Liu, S. X., Dakhovnik, A., Wandrey, F., Mozharovskyi, P., Zülli, F., & Ewald, C. Y. (2021). Youthful and age-related matreotypes predict drugs promoting longevity. Aging Cell, 20, e13441).

It was further found that the prolonged collagen homeostasis observed when supplementing the amino acids mentioned above in combination with each other corresponded to lifespan benefits. To this end, the lifespan of C. *elegans* fed with this combination of amino acids post-development starting from young adulthood was measured, and it was found that supplementation significantly increased lifespan by 6-27% across three independent trials. By contrast, supplementing these amino acids individually was not sufficient to increase lifespan.

To compare this increase in lifespan with collagen supplementation, *C. elegans* were fed with 400 pg/ml in food rat tail collagen and both conditions were assayed in parallel. It was found that collagen supplementation increased mildly the mean lifespan but more the maximum lifespan whereas supplementation with amino acids glycine, proline and hydroxyproline in combination with each other increased both mean and maximum lifespan. Thus, supplementation with the composition according to the present invention outperformed collagen supplementation for promoting longevity.

Therefore, the data clearly support that administering the composition of the present invention to the organism results in a prolongation of collagen homeostasis in the organism.

Specifically, a relevant prolongation in collagen homeostasis has been observed for a composition, in which the total amount of the combination of glycine, proline and hydroxyproline contained in the composition is at least 80 mg per kg body weight of the organism. Preferably, the total amount of the combination of glycine, proline and hydroxyproline is at least 90 mg, and most preferably is about 100 mg per kg body weight of the organism.

It is further preferred that each of glycine, proline and hydroxyproline are contained in an amount of at least 10 mg, preferably of at least 15 mg, per kg body weight of the organism.

As will be discussed in further detail below, the composition contains about 60 mg of glycine, about 20 mg of proline and about 20 mg of hydroxyproline per kg body weight of the individual, the efficacy of this composition being supported by data obtained in an aging mouse study.

As mentioned, glycine, proline and hydroxyproline are contained in the composition in free form, meaning that the amino acids are not present in the backbone of a peptide. The term "in free form" also encompasses the respective free amino acid in the form of a salt or ester, or the like.

The composition of the present invention encompasses both embodiments, in which besides glycine, proline and hydroxyproline one or more further amino acids are present, as well as embodiments in which glycine, proline and hydroxyproline are the sole amino acids contained. In this latter embodiment, the combination of amino acids contained in the composition is thus essentially consisting of glycine, proline and hydroxyproline.

In being directed to the non-therapeutic use for maintaining of prolonging collagen homeostasis in a living organism, the present invention is clearly different from the use of the compositions according to WO9527408A1 and EP0065462A1 discussed in the following:

WO9527408A1 relates to a sweetening agent comprising glycine, L-proline, and/or L-hydroxyproline. According to this document, the sweeting composition preferably comprises from 87.45% to 90% by weight of glycine, from 0.5% to 2% by weight of L-proline and from 0.5% to 2% of L-hydroxyproline. The composition is reported to have an anti-oxidation effect as well as aiding malnutrition and protein deficiency. In addition, WO 9527408A2 discloses that L-proline aids in the healing of intestinal diseases and burns and, further, is suitable for use as a protein supplement following surgery.

However, WO 9527408A2 does not contain any experimental data, which might support any of the beneficial effects mentioned in this document. In addition, no physiological explanation of the mechanisms underlying these effects is contained in WO 9527408A2. Even less is a potential effect on collagen homeostasis mentioned in this document.

A sweetening composition containing beta-alanine, glycine, tryptophan, proline, hydroxyproline and serine is further disclosed in EP0065462A1.

A potential effect on collagen homeostasis is not mentioned in this document either.

Since the use, to which the composition of the present invention relates, is disclosed neither in WO 9527408A2 nor in EP0065462A1, the subject matter of the invention is clearly different from both these documents.

As discussed above, the present inventors also found a correlation between the collagen homeostasis observed and an increase in the organism's lifespan. Thus, the composition of the present invention also relates to the non-therapeutic use in a method of increasing the healthspan and/or the lifespan of the organism.

More particularly, the present invention relates to the non-therapeutic use of the composition for maintaining or prolonging collagen homeostasis during aging of the organism. In this context, the term "aging" relates to the time-dependent progressive decline of physiological integrity leading to impaired function and increased vulnerability to death, as defined by C. Lopez-Otin et al. "Hallmarks of aging", Cell, 2013 Jun 6; 153(6): 1194-1217.

With regard to human individuals, persons at an age in the last quarter of the average lifespan, more particularly at an age of 70 years or older, are referred to as "elderly people" in the context of this invention.

Further, the term "extending the lifespan of a living organism" as used in the context of the present invention refers to a statistically significant extension of the lifespan over the average lifespan. The term "healthspan" as used in this context relates to the time spent in good health during the lifespan of an organism. More specifically, it relates to a statistically significant increase of pre-set parameters characterizing the organism's duration of the lifespan spent in good health and fitness.

Surprisingly, setting the ratio of amino acids fed to the organisms to an uneven distribution (i.e. a molar ratio differing from 1:1:1) led to an even more pronounced prolongation of col-144 expression during aging. This will be discussed in more detail by way of the specific working examples below, according to which an improvement in the prolongation of col-144 expression was observed for all compositions, in which glycine, proline and hydroxyproline are contained in a molar ratio differing from 1:1:1 (as compared to an administration of equal amounts of glycine, proline and hydroxyproline).

According to a particularly preferred embodiment of the composition, the glycine, proline and hydroxyproline are thus contained in a molar ratio differing from 1:1:1.

As will also be discussed by way of the specific working examples, the molar ratio of glycine to proline is preferably in the range from 10:1 to 1:3, more preferably from 6:1 to 1:2, even more preferably from 4:1 to 2:1, and most preferably is about 3:1. Within this ratio, a highly beneficial effect in terms of the mean lifespan of the organism has been observed.

With regard to the molar ratio of hydroxyproline to proline, it is preferred that this is from 3:1 to 1:3, more preferably from 2:1 to 1:2.

A particularly pronounced effect in terms of increase of the organism's lifespan has been observed for a composition, in which the molar ratio of glycine to proline to hydroxyproline is about 10:1:1.

In addition, very pronounced effects regarding collagen homeostasis and ultimately an increase in healthspan and lifespan has also been observed for a molar ratio of glycine to proline to hydroxyproline of about 3:1:1. This effect has been confirmed in the C. elegans model, in which a composition containing 15 mM glycine, 5 mM proline and 5 mM hydroxyproline was used, as well as in the human skin cell (fibroblast) culture using a composition containing 3 mM glycine, 1 mM proline and 1 mM hydroxyproline.

As mentioned, the data showing the lifespan increase discussed above were obtained by tests performed on solid media, where *C. elegans* are typically less motivated to move and rest in the food during aging (Statzer C, Reichert P, Dual J & Ewald CY (2022) Longevity interventions temporally scale healthspan in Caenorhabditis elegans. iScience 24, 103983). To measure their active movement capability during aging (i.e., healthspan), their thrashing rates in liquids (i.e., swimming) during their entire lifespan were assayed. It was found that all ratios showed prolonged movement capability during aging at least in one out of two trials. Administration of a composition with a ratio Gly:Pro:Hyp of 3:1:1 (in the following referred to as [3 Gly : 1 Pro : 1 Hyp]) consistently improved activity during aging. In one trial, the only two combinations showing higher swimming rates during mid-life than [3 Gly : 1 Pro : 1 Hyp] were [3 Gly : 1 Pro : 0 Hyp] and [1 Gly : 1 Pro : 3 Hyp]. At the very old age of >24 days of adulthood at the end of the lifespan, [3 Gly : 1 Pro : 1 Hyp] fed C. *elegans* were the most active, further supporting the particular relevance of this composition. Taken together the data are in clear support that a combined administration of amino acids Gly, Pro and Hyp has synergistic effects that are more than just the sum of its parts.

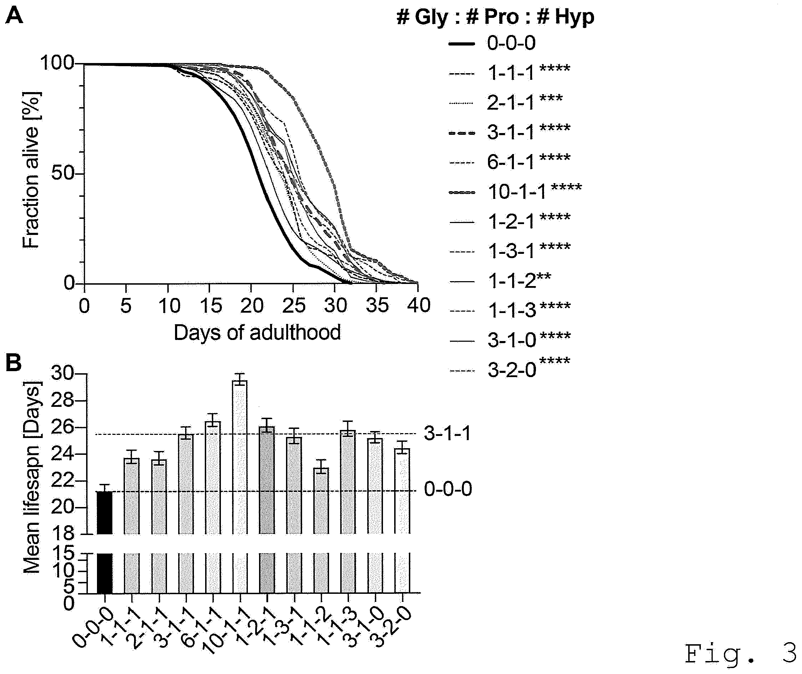

According to a further preferred embodiment, the composition further contains alpha-ketoglutarate, since it was found that supplementing C. elegans with alpha-ketoglutarate from young adulthood increased their lifespan, in addition to the longevity-induced effects of the specific amino acid combination discussed above. To assess whether the longevity upon alpha-ketoglutarate supplementation would be mediated through its co-factor activity for prolyl-hydroxylase, the present inventors used a mutant dpy-18(ok162) of the prolyl-4-hydroxylase alpha subunit that eliminates its propyl-4-hydroxylase activity (as reported by Friedman L, Higgin JJ, Moulder G, Barstead R, Raines RT & Kimble J (2000) Prolyl 4-hydroxylase is required for viability and morphogenesis in Caenorhabditis elegans. P Natl Acad Sci USA 97, 4731-41). It was found that the longevity upon alpha-ketoglutarate supplementation is completely abolished in dpy-18(ok162) mutants, suggesting that prolyl-hydroxylase activity is required for alpha-ketoglutarate-induced longevity. Intriguingly, [3 Gly : 1 Pro : 1 Hyp] supplementation was still sufficient to increase lifespan, suggesting bypassing the requirements of prolyl-hydroxylase activity for longevity (Figure 3d-f, Supplementary Figure 3a-c, Supplementary Table 2) . These results are in clear support that alpha-ketoglutarate and [3 Gly : 1 Pro : 1 Hyp] work through parallel pathways for their additive and synergistic effects on promoting healthy aging.

To gain mechanistic insights into how supplementation of the amino acid combination of the present invention in general, and [3 Gly : 1 Pro : 1 Hyp] in specific, affects human cells *in vitro,* RNA sequencing upon 2h, 8h, and 24h of incubation of human dermal fibroblasts with [3 Gly : 1 Pro : 1 Hyp] was performed. Gene set enrichment analysis (GSEA) showed that at 8h and 24h the most significantly enriched gene sets with positive enrichment scores were involved in collagen and extracellular matrix homeostasis including "extracellular matrix structural constituent" and "collagen-containing extracellular matrix". The most significantly enriched gene sets with negative enrichment scores primarily involved mitochondrial and ribosomal processes and included "mitochondrial inner membrane" and "ribosomal subunit". The overall predominant signature from the GSEA was an upregulation in collagen and extracellular matrix-associated processes, a prominent downregulation of ribosomal components, and a less robust downregulation of mitochondrial-associated pathways. The upregulation of collagen-related processes was the most robust signature in the dataset, with a highly consistent increase in expression of most extracellular matrix-associated genes at the 8 and 24-hour time points. This trend was also evident when the 'matrisome', or set of genes associated with extracellular matrix structure and remodeling processes, was assessed. Overall upregulation was observed across all matrisome domains but was particularly pronounced in collagens. Upregulation was also seen in glycoproteins and genes involved in extracellular matrix remodeling processes.

At 8h and 24h, the strongest upregulated gene signatures were extracellular matrix genes, cell-cell adhesion, mitochondrial and ribosomal components. Except for mitochondria, all these gene enrichments point towards extracellular matrix (ECM) remodeling.

According to a particularly preferred embodiment, the present invention relates to the non-therapeutic use described above in a mammal, and more particularly in a human individual.

More preferably, the individual subject to the non-therapeutic use is an infant, a growing-up child, an athlete or an elderly person, in particular an elderly person. However, other mammals are also encompassed by this preferred embodiment, in particular pets, such as dogs or cats.

A particularly efficient and long-lasting effect in terms of maintaining or prolonging collagen homeostasis, and ultimately in increasing the individual's lifespan and/or healthspan, has been shown for a use in which the composition is administered daily over a period of at least 4 months, more preferably at least 6 months. Specifically, the effect was confirmed experimentally in very old mice (of 20 months of age).

According to a further aspect, the present invention also relates to a nutritional supplement preparation for a mammal, the preparation comprising the composition as described above, wherein a single dosage unit of the preparation contains at least 80 mg of the combination of glycine, proline and hydroxyproline per kg body weight of the individual.

As discussed above, the nutritional supplement preparation is primarily for a human individual, but can also be administered to other mammals, in particular pets, such as dogs or cats.

As also discussed above, the total amount of the combination of glycine, proline and hydroxyproline contained in the composition is preferably at least 90 mg, and most preferably about 100 mg per kg body weight of the organism.

According to a particularly preferred embodiment, the single dosage unit contains about 60 mg of glycine, about 20 mg of proline and about 20 mg of hydroxyproline per kg body weight of the individual. In as far as the nutritional supplement preparation is for a human individual, the total dosage of glycine should not exceed 5 g, irrespective of the individual's body weight.

A highly preferred nutritional supplement preparation contains a single dosage unit of about 5 g glycine, 1.67 g of proline and 1.67 g of hydroxproline in total. For a female having an average body weight of about 60 kg, this corresponds to a single dosage unit of 83.3 mg glycine, 27.8 mg proline, and 27.8 mg hydroxyproline per kg body weight, whereas for a male having an average body weight of about 80 kg, this amounts to a single dosage unit of 62.5 mg glycine, 20.8 mg proline and 20.8 mg hydroxyproline per kg body weight.

According to a preferred embodiment of this further aspect of the invention, the nutritional supplement preparation is an oral preparation, in particular in the form of a powder or a tablet.

It is further preferred that the preparation contains at least one further ingredient selected from the group of vitamins, in particular vitamin C, cofactors, lipids, carbohydrates, carotinoids, in particular astaxanthin, and combinations thereof.

Alternatively to the non-therapeutic use described above, the composition can according to a further aspect be used therapeutically, more specifically in a method of treating a disease associated with the malfunction of collagen homeostasis in a mammal, in particular a human individual, during aging, the composition being as defined above. More specifically, the composition can be used in a method of treating frailty, in particular in an elderly person, more particularly in a person of at least 70 years of age.

### EXAMPLES

### Materials and Methods

### C. elegans strains

For the experiments referred to in the following, the following strains of C. elegans were used:
- Wild type N2
- LSD2002 spe-9(hc88) I; xchIs001 [Pcol-144::GFP; pha-1(+)] X
- TJ1060 spe-9(hc88) I; rrf-3(b26) II.
- JK2729 dpy-18(ok162) III

### Supplementation of amino acids for collagens reporter read out in C. elegans

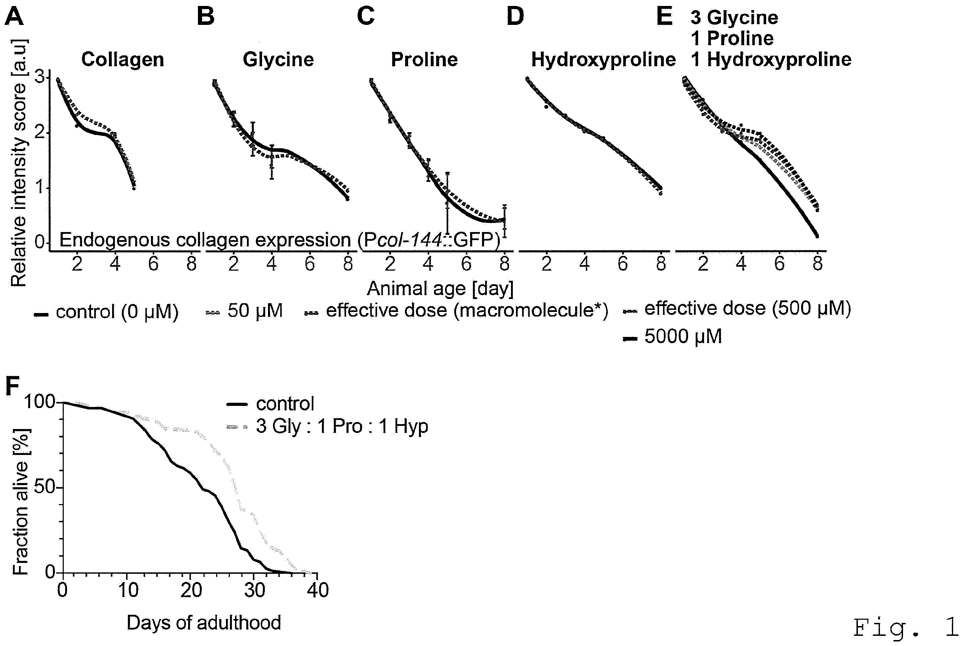

For determining the most effective amino acid ratios (Fig.1 a-e, and S.1 c-f) 0.1 M stocks of glycine (Sigma-G7126), proline (Sigma-P5607), and hydroxyproline (Sigma56250) were prepared in filter-sterilized via millipore 0.2 µm pore membrane (SARSTEDT-83.1826.001) mQ-H₂O. Afterward, these stocks were added into NGM agar during preparation (with either carbenicillin or ampicillin, and nystatin, see WormBook nematode maintenance protocols), and mixed thoroughly prior to solidifying. Thus, correspondent amino acids concentration were achieved: either 50 µM, 500 µM, or 5000 µM. Finally, 5 mM corresponds to "one ratio point" later (3-1-1 = 15 mM glycine - 5 mM proline - 5 mM hydroxyproline). After determining 1 ratio point, all further experiments were done in the same way, except for stocks, where amino acids solubilized in mQ-H2O to their maximal solubility points: 4 M for glycine, 3.17 M for proline, and 198 mM for hydroxyproline correspondingly. This was done to decrease excessive dilution of NGM with water especially in experiments with 10-1-1, 1-1-2, and 1-1-3 ratios, to avoid high salt disbalance in nematode growth media. Control and amino acids containing plates were kept at room temperature for 2-3 days to dry after preparation. Fresh OP50 bacteria were grown a day before the experiment to achieve their stationary phase (8 h or overnight). Then heat-inactivated (to exclude 21 metabolizing amino acids by bacterial cells) at 60°C for a minimum of 45 min, cooled down to RT. Then, concentrated 10-fold with 2809 rcf / 15' centrifugation and seeded onto plates after resuspending bacterial pellet. 1 ml, 300 µl, or 120 µl of bacterial concentrate were added to 15 cm, 6 cm, or 3.5 cm plates, respectively. Plates were ready to use once NGM absorbed all the excess liquid after seeding. The quantification was performed according to Statzer et al., 2021 (Statzer et al. 2021).

### Lifespan assays

Manual lifespan assays were performed at 20°C on heat-inactivated OP50 bacteria, seeded onto NGM plates, containing nystatin if not stated otherwise. N2 and JK2729 worms were grown until the L4 stage on plates with heat-inactivated OP50, then transferred to 50 µM FUdR containing plates to prevent progeny development. On other hand, TJ1060 and LSD2002 worms were kept at 25°C until the young adulthood stage, the restrictive temperature for spe-9(hc88) temperature-sensitive mutation and then, after confirmation of the C. elegans' sterility, re-picked to plates with correspondent conditions, stated in experiments. C. elegans were observed/counted every second day until AD12, with daily quantification later on, until the end of the experiment. All animals that dried on plate walls, deeply dug into agar, as well as C. elegans with protruding vulva and matricide phenotype were documented as censored for further analysis. Calculations of manual lifespans data were performed using the Kaplan-Meier estimator in GraphPad Prism^{™} 8.0.1 software. Log-rank (Mantel-Cox) statistical analysis was used for the significance determination. Mean lifespan changes were calculated separately with t-test or One-way ANOVA and SEM (if not stated otherwise) utilization for statistics in the same software package.

### Longitudinal movement assays

First, the population of 2000-5000 worms was synchronized at AD1-AD4 and bleached (for details of the method, see Teuscher AC, Statzer C, Pantasis S, Bordoli MR & Ewald CY (2019) Assessing Collagen Deposition During Aging in Mammalian Tissue and in Caenorhabditis elegans. Methods Mol Biology Clifton N J 1944, 169-188*).* 1 µl of ampicillin (100 mg/mL) and 0.5 µl of tetracycline (100 mg/mL) per 1 ml of buffer with bleached animals were added. Animals were left overnight for hatching and L1-synchronization.

Second, the synchronous L1 populations of C. elegans were filtered via 11.0 µm pore size, hydrophilic nylon membrane (Millipore - NY1104700). The clean population of L1s was counted under a stereomicroscope in 3 x 10 µl drops, to define C. elegans concentration. Meanwhile, 50 ml of stationary-phase fresh heat-inactivated OP50 bacteria were concentrated and all LB-media leftovers were discarded. Then, the bacterial pellet was resuspended thoroughly in 10 ml of S-complete buffer in aseptic conditions < 10 cm near a gas burner. Note that all the pipets and pipet-boys were sprayed with 70% ethanol prior to the experimental procedure.

3000-4000 animals from the filtered L1 populations were concentrated at 1800 rcf / 1' in 15 ml Falcon^{®}, and M9 buffer was discarded until almost a packed worm pellet was reached. Afterward, 10 ml of S-complete with food was added to worms and wellmixed. Later, this tube was sprayed with 70% ethanol and transferred under a sterile laminar-flow hood, pouring out into a 10 cm sterilized tray. Using a multichannel pipette 100 µl of buffer with worms was resuspended and added into the U-bottom 96-well plate per well, achieving ~ 30-40 animals per well.

Plates with worms were left in small sealed plastic bags at 20°C (or 25°C for TJ1060) on a working orbital shaker (450-500 rpm) for internal aeration. Animals were checked every day for the presence of contaminations and abnormalities with growth and development. After reaching the L4 stage 100 µM FUdR of final concentration was added for N2 worms. Correspondent amino acids were added at AD1 in mentioned concentrations. Animals were fed with additional heat-inactivated bacteria (but 50% of the initial amount) every 5th day until AD15 when they exhibit a drastic drop in food consumption.

Motility (fitness) measurement was performed using WMicrotracker ONE ^{®} (from InVivo Biosystems^{™}). For measurements, we used 3 x 30 min "time-buckets", and the mean value was calculated for each well, in turn, we utilized 8 wells per condition to have a robust quantification. XY-graphs (health-span curves) were built in GraphPad Prism^{™} 8.0.1 software for absolute motility readings. The area under the curve (AUC) for descriptive statistics and the two-way ANOVA test were used to calculate the significance between curves, and mean values of 8 wells of the same condition were used as reference points.

### Cultivation and 3 Gly : 1 Pro : 1 Hyp supplementation of human dermal fibroblasts

Human dermal fibroblasts (HDF, neonatal, Sigma-Aldrich, SKU 106-05N) were grown in a fibroblast growth medium (Sigma-Aldrich, SKU 116-500). Prior to 3-1-1 supplementation, cells were harvested using accutase cell detachment solution (w: 0.5 mM EDTA, w: Phenol red; Pan-Biotech; P10-21500) and centrifuged (200 g, 5 min, RT). Cells were resuspended in fibroblast growth medium without supplementation or with supplementation of a mixture of 3 mM glycine, 1 mM proline, and 1 mM hydroxyproline (sterile filtered). Subsequently, HDFs were seeded in 10 cm plates (30% confluency). Cells were cultivated for 2 h, 8 h, and 24 h and lysed on the plate using 1 ml TRIzol-Reagent (ambion^{®}, ref# 15596018). Cells harvested at 0 h were centrifuged again (200 g, 5 min, RT) immediately after resuspension and lysed using 1 ml Trizol. RNA extraction was performed using a standard phenol-chloroform method. Briefly, 1mL Trizol lysate was combined with 0.2mL chloroform, mixed, and centrifuged (10,000 x g, 10min, 4C). The upper layer (~0.4mL) was taken and combined with 0.5mL of isopropyl alcohol, mixed, and centrifuged (10,000 x g, 10min, 4C). The supernatant was discarded and the pellet was washed twice with 80% ethanol, dried, and resuspended in RNase-free water.

### RNA sequencing

RNA quality was confirmed using a NanoDrop 1000 spectrophotometer and an Agilent 2100 Bioanalyzer. cDNA libraries were prepared using the Illumina TruSeq Stranded Total RNA Sample Preparation protocol. 150bp paired-end reads were generated on an Illumina NovaSeq with a read depth of 20 million paired reads per sample. Reads were aligned and annotated to the Ensembl Homo sapiens GRC38.p13 reference genome and associated GTF annotation file using the align and featureCounts functions from the Rsubread package (2.10.4) in R (4.2.0) (Liao Y, Smyth GK & Shi W (2019) The R package Rsubread is easier, faster, cheaper and better for alignment and quantification of RNA sequencing reads. Nucleic Acids Res 47, e47-e47*).* Differential gene expression analysis was performed using the voom and eBayes functions from the EdgeR (3.38.1) and Limma (3.52.2) packages (Ritchie ME, Phipson B, Wu D. Hu Y, Law CW, Shi W & Smyth GK (2014) limma powers differential expression analyses for RNA-sequencing and microarray studies. Nucleic Acids Res 43, e47*;* Robinson MD, McCarthy DJ & Smyth GK (2010) edgeR: a Bioconductor package for differential expression analysis of digital gene expression data. Bioinformatics 26, 139-140). Gene set enrichment analysis was performed using the gseGO function from the clusterProfiler packages (4.4.4) (Wu T, Hu E, Xu S, Chen M, Guo P, Dai Z, Feng T, Zhou L, Tang W, Zhan L, Fu X, Liu S, Bo X & Yu G (2021) clusterProfiler 4.0: A universal enrichment tool for interpreting omics data. Innovation 2, 100141)*.*

### Mouse Study

C57BL/6J mice were ordered from Janvier between the ages of 18 and 21 months, which is the equivalent of approximately 55 to 60 human years of age. All ordered mice were included in the study, except for the ones that reached termination criteria before the start of the study, as defined in the animal license. Due to a supply shortage of male mice because of the COVID-19 pandemic, the first cohort consisted of 100 females and only 20 males. However, 40 additional males could be obtained at later time points, and were analyzed in two additional cohorts of 20 mice each. Cohort two was started six weeks after cohort one, while cohort three was started 13 weeks after cohort one.

### Mice, husbandry, and diets

The animal rooms were kept at 20-22° C and 50-70% humidity. The mice were held in individually ventilated cages where the 12:12h light-dark cycle can be adapted, with the dark period being during the day from 8am to 8pm. This allowed us to perform all measurements when the mice were in their active phase. Up to four mice were housed in one cage with ad libitum access to food and autoclaved drinking water. In case of the death of a cage mate or fighting between males, the mice were singly housed, which is justified in the animal license and approved by the Kanton Zurich. All animals were provided enrichment, including houses, tissues, and chew blocks. Mice cages (including water bottles and enrichment) were changed weekly in a laminar flow hood and bodyweight and food intake was recorded for all mice. Prior to starting the study, all mice were fed a standard AIN-93G based diet84 for up to one month during which time the baseline measurements were performed. All study diets were obtained from a commercial vendor (Research Diets, New Brunswick NJ).

### Geroprotectors

Geroprotectors were delivered to the mice via the food. The compounds were formulated into the base diet (AIN-93G) by Research Diets. Weekly food consumption and body weight were monitored during the study period. The following geroprotectors were included in this study: (1) drug 3 Gly : 1 Pro : 1 Hyp 100mg/kg, a proprietary combination of amino acids (each of which obtained individually from Sigma-Aldrich, St. Louis MO).

### Blinding

Study diets were coded at the time of manufacture by the commercial diet supplier (Research Diets, New Brunswick NJ) to blind experimenters. For data analysis for this thesis, the diets were unblinded after the data collection of the three-month time point to allow for correct data analysis. The limitations of this approach are discussed in the section "Study limitations".

### Monitoring the animal welfare

Mice underwent daily monitoring during the study according to the parameters set out in Appendix 4. Mice reaching termination criteria were euthanized according to approved protocols.

### Experimental design

After an acclimatization period (up to one month), female (n=100) and male (n=60) C57BL/6J mice between the ages of 18 to 21 months were distributed to cages with two to four animals of the same sex per cage, matched for the same mean bodyweight per cage. The cages were then randomly allocated to the five experimental groups using a random number generator, with n=10-20 animals of each sex per intervention. The decodes for the diets (coded at the manufacturer) were kept by the study investigator until the end of the study, which allowed for the blinding of all experimenters. At the beginning of the six-month study period, baseline measurements were performed to assess the health of the mice, with the mFI as the primary study outcome. Additional health parameters included as secondary outcomes are bodyweight and body composition, food intake, hematology analysis, blood and serum markers, electrocardiogram to measure heart rate and heart rate variability, grip strength, open field test, microbiome composition with 16s RNA sequencing on faecal samples, and urinary metabolome analysis.

### Results

The results of the experiments are discussed in the following, referring to the attached figures, of which:
- Fig. 1: shows the relative intensity score of endogenous collagen expression (Pcol-144::GFP) after supplementing with collagen (A), glycine (B), proline (C), hydroxyproline (D) and a composition of the present invention (3 Gly : 1 Pro : 1 Hyp) as a function of animal age, as well as survival curves obtained by supplementation of [3 Gly : 1 Pro : 1 Hyp] to LSD2002 Pcol-144::GFP C. *elegans* as compared to the respective control experiment;

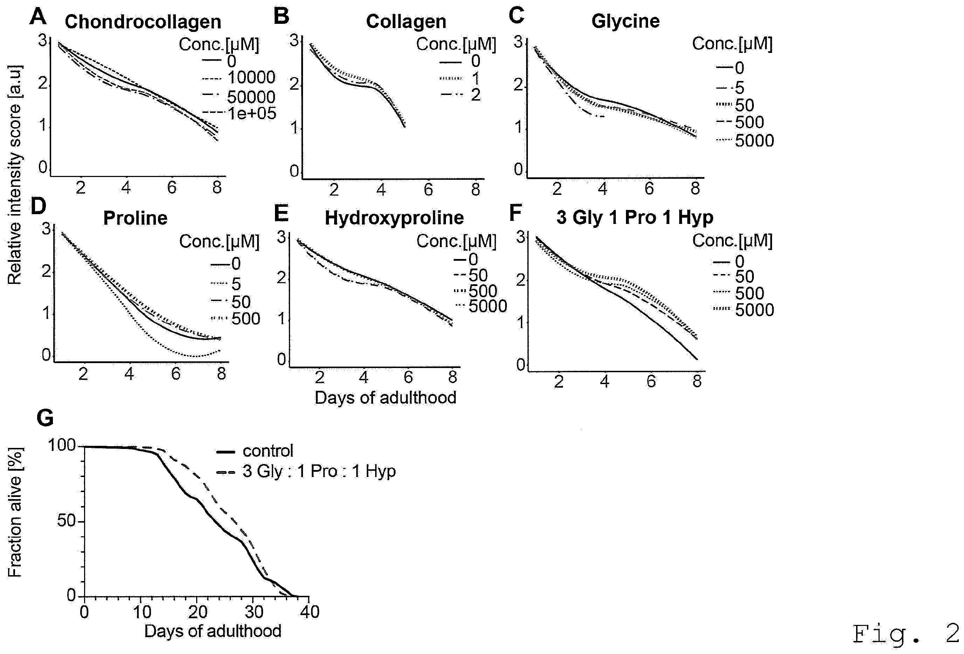

- Fig. 2: shows the relative intensity score of endogenous collagen expression (Pcol-144::GFP) after supplementing with chondrocollagen (A), rat tail collagen (B), glycine (C), proline (D), hydroxyproline (E), or a composition of the present invention [3 Gly : 1 Pro : 1 Hyp] (F) in different concentrations as a function of animal age, as well as the survival curve (G) obtained by supplementation of [3 Gly : 1 Pro : 1 Hyp] to temperature-sensitive wild-type background TJ1060 *C*. *elegans* as compared to the respective control experiment;
- Fig. 3: shows the fraction of C. *elegans* alive as a function of days of adulthood after supplementation with the combination of amino acids according to the present in different ratios as compared to the respective control experiment (A) as well as a representation of the mean lifespan of the organisms after the supplementation (B);

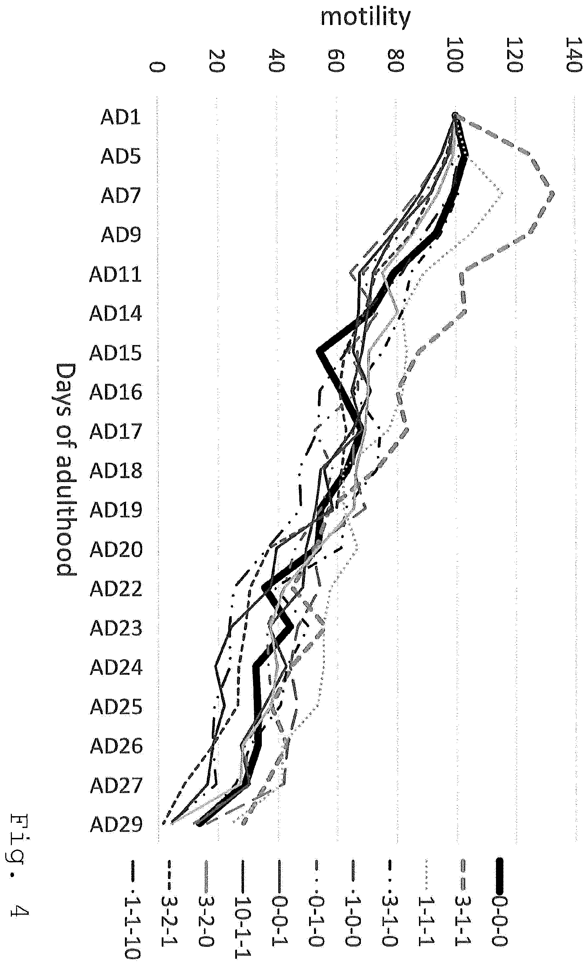

- Fig. 4: shows the longitudinal motility of C. *elegans* alive as a function of days of adulthood after supplementation with the combination of amino acids according to the present in different ratios as compared to the respective control experiment;

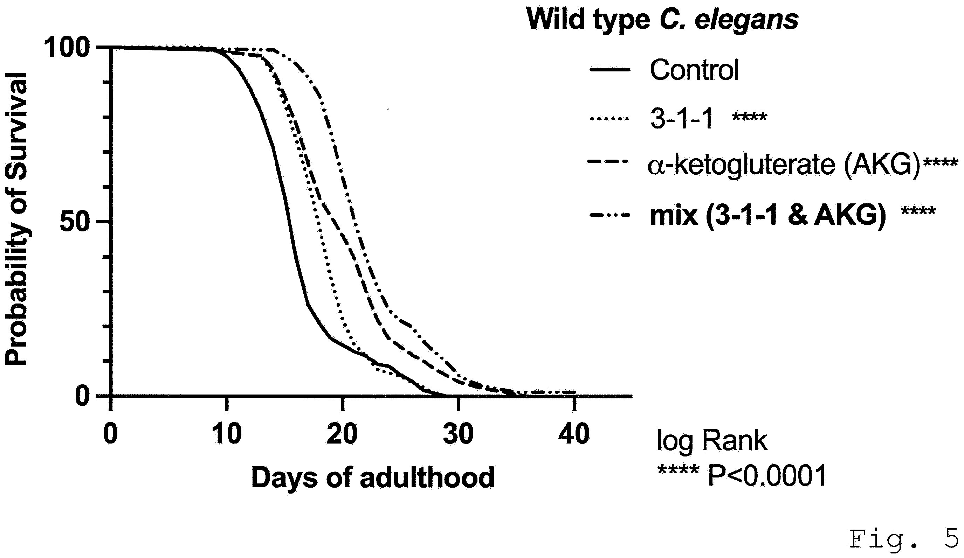

- Fig. 5: shows the fraction of C. *elegans* alive as a function of days of adulthood after supplementation with the combination of amino acids according to the present, alpha-ketoglutarate and a mixture of the amino acid combination with alpha-ketoglutarate, as compared to the respective control experiment;

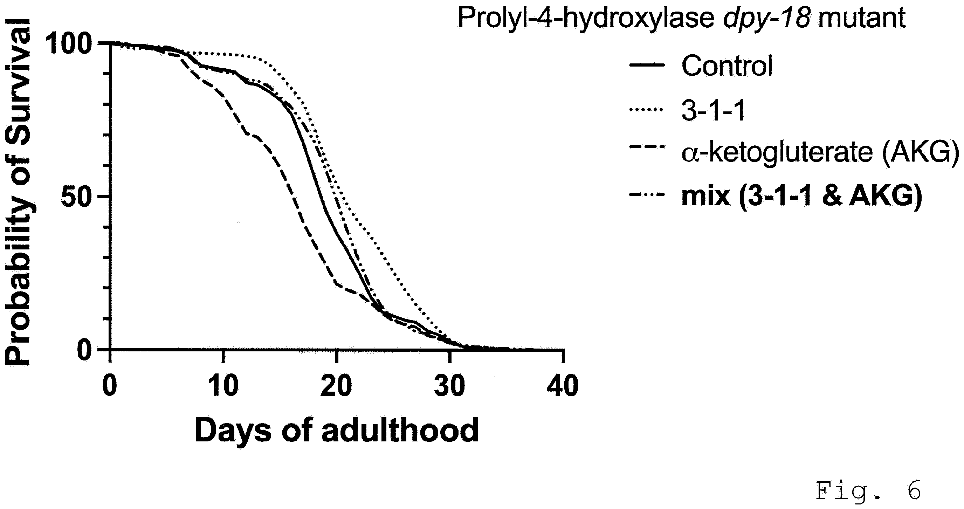

- Fig. 6: shows the fraction of C. *elegans* alive as a function of days of adulthood after supplementation as according to Fig. 5 but using a mutant dpy-18(ok162) of the prolyl-4-hydroxylase alpha subunit;

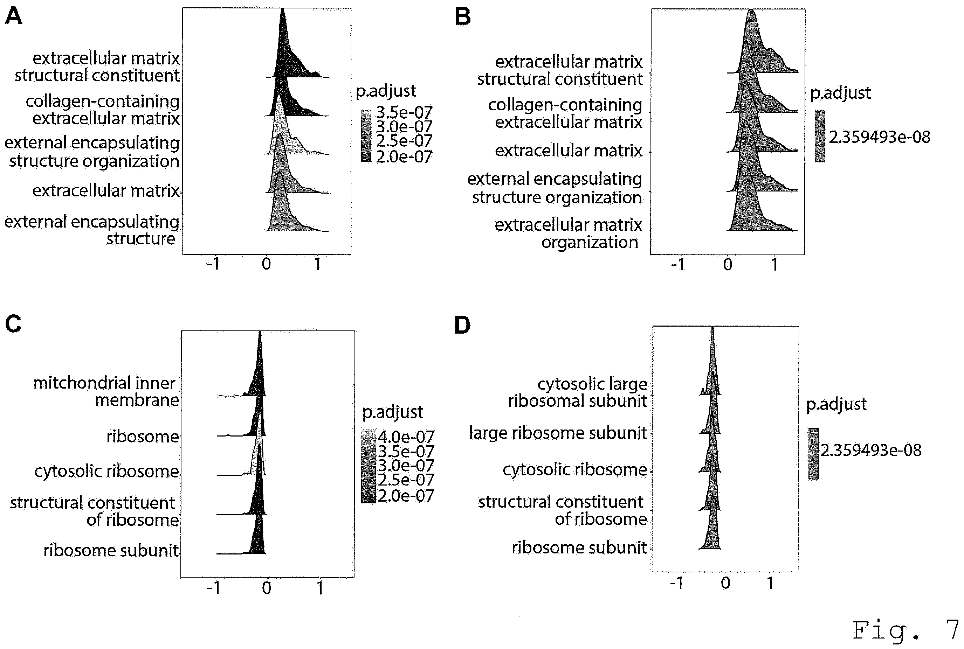

- Fig. 7: shows the gene set enrichment analysis (GSEA) data obtained by RNA sequencing upon 2h, 8h, and 24h of incubation of human dermal fibroblasts with [3 Gly : 1 Pro : 1 Hyp], with the top 5 GO pathways with positive enrichment scores at 8 hours (A) and 24 hours of treatment (B) being shown; and

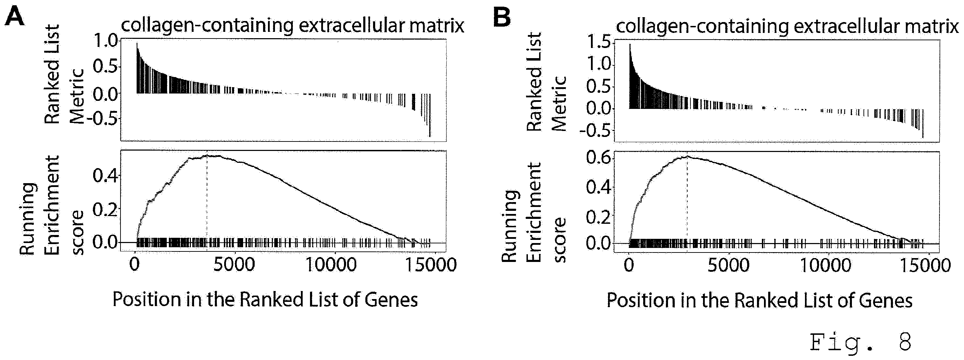

- Fig. 8: shows the gene set enrichment plot for the GO collagen-containing extracellular matrix pathway at 8 hours (A) and 24 hours of treatment (B).

Referring to Fig. 1-A to 1-E, synchronized L4 transgenic C. *elegans* driving GFP with the collagen col-144 promoter (LSD2002 Pcol-144::GFP) were placed on culturing plates containing heat-killed OP50 bacteria food with either rat tail collagen (A), glycine (B), proline (C), hydroxyproline (D), or a mixture of [3 Gly : 1 Pro : 1 Hyp] (E), and GFP intensity was scored during aging at 25°C.

As shown in Fig. 1-F representing the respective survival curves, supplementation of [3 Gly : 1 Pro : 1 Hyp] starting from young adulthood increased the lifespan of LSD2002 Pcol-144::GFP C. *elegans* as compared to the respective control experiment (with supplementation of the composition of the present invention).

Referring to Fig. 2-A to 2-F, synchronized L4 transgenic C. *elegans* driving GFP with the collagen col-144 promoter (LSD2002 Pcol-144::GFP) were placed on culturing plates containing heat-killed OP50 bacteria food with different concentrations of either chondrocollagen (A), rat tail collagen (B), glycine (C), proline (D), hydroxyproline (E), or a composition of the present invention [3 Gly : 1 Pro : 1 Hyp] (F), and GFP intensity was scored during aging at 25°C. As shown by the respective survival curve represented by Fig. 1-G, supplementation of [3 Gly : 1 Pro : 1 Hyp] starting from young adulthood increased the lifespan of temperature-sensitive wild-type background TJ1060 C. *elegans.* (Due to the increase of mean but not maximum increase in lifespan in this trial, a Gehan-Breslow-Wilcoxon test was used to determine significance.

As discussed above, the active movement capability of C. *elegans,* reflected by their thrashing rates in liquids (i.e., swimming) was prolonged during aging of at least in one out of two trials. Administration of a composition with a composition of [3 Gly : 1 Pro : 1 Hyp]) consistently improved activity during aging.

Referring to Fig. 5, supplementing C. *elegans* with alpha-ketoglutarate from young adulthood increased their lifespan, in addition to the longevity-induced effects of the specific amino acid combination discussed above. As shown in Fig. 6 and as also discussed above, the longevity upon alpha-ketoglutarate supplementation is completely abolished in dpy-18(ok162) mutant JK2729 (eliminating its propyl-4-hydroxylase activity), but [3 Gly : 1 Pro : 1 Hyp] supplementation was still sufficient to increase lifespan of this mutant.

The gene set enrichment analysis (GSEA) represented in Fig. 7 showed that at 8h and 24h the most significantly enriched gene sets with positive enrichment scores were involved in collagen and extracellular matrix homeostasis including "extracellular matrix structural constituent" and "collagen-containing extracellular matrix", as discussed above (Fig. 7-A and 7-B). As also discussed above, the most significantly enriched gene sets with negative enrichment scores primarily involved mitochondrial and ribosomal processes and included "mitochondrial inner membrane" and "ribosomal subunit" (Fig. 7-C and 7-D).

According to the network plots of the gene set enrichment analysis results after 8 hours and 24 hours of treatment (not shown), the overall predominant signature from the GSEA was an upregulation in collagen and extracellular matrix-associated processes, a prominent downregulation of ribosomal components, and a less robust downregulation of mitochondrial-associated pathways.

The upregulation of collagen-related processes was the most robust signature in the dataset, with a highly consistent increase in expression of most extracellular matrix-associated genes at the 8 and 24-hour time points, as shown in Fig. 8-A and 8-B, respectively). This trend was also evident when the ^matrisome', or set of genes associated with extracellular matrix structure and remodeling processes, was assessed. Overall upregulation was observed across all matrisome domains but was particularly pronounced in collagens. Upregulation was also seen in glycoproteins and genes involved in extracellular matrix remodeling processes.

At 8h and 24h, the strongest upregulated gene signatures were extracellular matrix genes, cell-cell adhesion, mitochondrial and ribosomal components. Except for mitochondria, all these gene enrichments point towards extracellular matrix (ECM) remodeling.

## Claims

1. Non-therapeutic use of a composition for maintaining or prolonging collagen homeostasis in a living organism and/or for extending the healthspan and/or lifespan of a living organism, the composition containing glycine, proline and hydroxyproline in free form.

2. Use according to claim 1, the total amount of the combination of glycine, proline and hydroxyproline contained in the composition being at least 80 mg, preferably at least 90 mg, and most preferably about 100 mg per kg body weight of the organism.

3. Use according to claim 1 or 2, each of the glycine, proline and hydroxyproline being contained in an amount of at least 10 mg, preferably of at least 15 mg, per kg body weight of the organism.

4. Use according to any of the preceding claims, wherein the glycine, proline and hydroxyproline are contained in a molar ratio differing from 1:1:1.

5. Use according to any of the preceding claims, wherein the molar ratio of glycine to proline is in the range from 10:1 to 1:3, preferably from 6:1 to 1:2, more preferably from 4:1 to 2:1, and most preferably is about 3:1.

6. Use according to any of the preceding claims, wherein the molar ratio of hydroxyproline to proline is from 3:1 to 1:3, preferably from 2:1 to 1:2.

7. Use according to any of the preceding claims, wherein the molar ratio of glycine to proline to hydroxyproline is about 10:1:1.

8. Use according to any of the preceding claims, wherein the composition further contains alpha-ketoglutarate.

9. Use according to any of the preceding claims, wherein the organism is a mammal, in particular a human individual.

10. Use according to claim 9, wherein the human individual is an infant, a growing-up child, an athlete or an elderly person, in particular an elderly person.

11. Use according to any of claims 9 to 10, the composition being administered daily over a period of at least 4 months, more preferably at least 6 months.

12. Nutritional supplement preparation for a mammal, in particular a human individual, the preparation comprising the composition as defined in any of the preceding claims, wherein a single dosage unit of the preparation contains at least 80 mg of the combination of glycine, proline and hydroxyproline per kg body weight of the individual.

13. Nutritional supplement preparation according to claim 12, wherein the single dosage unit contains about 60 mg of glycine, about 20 mg of proline and about 20 mg of hydroxyproline per kg body weight of the individual.

14. Nutritional supplement preparation according to claim 12 or 13, the preparation being an oral preparation, in particular in the form of a powder or a tablet.

15. Nutritional supplement preparation according to any of claims 12 to 14, the preparation containing at least one further ingredient selected from the group of vitamins, in particular vitamin C, cofactors, lipids, carbohydrates, carotinoids, in particular astaxanthinand combinations thereof.
